# EUROPEAN PATENT APPLICATION

(11) **EP 4 483 778 A1**
(43) Date of publication of application: **01.01.2025**
(21) Application number: 24160240.8
(22) Date of filing: 28.02.2024
(51) Int. Cl.: A61B 1/00

(54) **HANDLE FOR AN ENDOSCOPE**

(30) Priority: 28.06.2023 EP 23182137; 05.09.2023 EP 23195491; 05.09.2023 EP 23195488
(71) Applicant: Ambu A/S, 2750 Ballerup (DK)
(72) Inventor: KLÜVER, Anders, 2750 Ballerup (DK); JENSEN, Thomas Bachgaard, 3500 Værløse (DK)
(74) Representative: Winter, Brandl - Partnerschaft mbB

(57) **Abstract**

Endoscope (1) comprising a handle (2) assembled by application of fasteners (17) being integral with a shell part (12), the assembled shell parts (12) form a compartment (36). The handle comprises at least one component selected from the group: a roller (28) for bending a bending section (20) by pulling a steering wire (55), a wire pipe fastener (34) connecting wire pipes (56) to a handle shell (12), an enclosure (32) for a printed circuit board. The handle (2) comprises interfaces (21) configured to secure the at least one component to a shell part (12) inside the compartment (36), the interfaces (21) are integral with a shell part (12). The shell parts are made from polypropylene, wherein the polypropylene comprises bio-based polypropylene.

## Description

### Technical Field

The present teaching relates to an endoscope comprising a handle including handle shells.

### Background

The application of single-use endoscopes for medical procedures have become widespread due to the eliminated risk for cross contamination and the improved availability at hospitals where time consuming cleaning procedures of re-usable endoscopes are avoided.

Single-use endoscopes are often made with a relatively high content of polymers and assembled by use of gluing processes. This keeps manufacturing costs low.

The increasing concern for environmental impact and climate change has resulted in a focus on reducing the carbon dioxide footprint from single-use products.

### Summary

In a first aspect, the present teaching provides an endoscope comprising a handle for controlling the endoscope. The handle is assembled from handle shells by fasteners integrated with the handle shells. The handle shell parts comprise polypropylene (PP). The polypropylene content may comprise virgin and recycled polypropylene, which may be bio-based and/or carbon based.

In one embodiment the present teaching relates to an endoscope comprising a handle and an insertion cord with a bending section which is bendable by manipulation of steering wires movably arranged in wire pipes. The handle comprises two shell parts assembled with fasteners. Each of the fasteners is made of the same material and in one-piece with one of the two shell parts. The assembled shell parts form a compartment. The handle also comprises at least one component and an interface configured to secure the at least one component to a shell part inside the compartment. Part of the interface is made in one-piece with, and of the same material as, a shell part. The interface extends from the shell part into the compartment. Further, the shell parts comprise polypropylene, where the PP may comprise bio-based PP.

In one variation, the at least one component is selected from the group comprising: a roller for bending the bending section by pulling a steering wire, a wire pipe fastener connecting the wire pipes to a handle shell, or an enclosure for a printed circuit board.

It has been found that this embodiment can provide a single-use endoscope having a lower carbon dioxide footprint. The use of PP for the handle shell parts has not previously been considered because gluing on PP is difficult. PP has a low surface energy - typically only 29mN/m. To achieve "wetting" or adhesion, with most adhesives surface energy above 36mN/m is needed. High surface energy plastics, such as Acrylonitrile Butadiene Styrene (ABS) and polycarbonate, bond well with standard conventional adhesives and tapes possessing lower surface energy. But the use of PP for the shell parts will reduce the carbon dioxide footprint compared to the use of ABS. When part of the PP is bio-based, i.e., originates from renewable feedstocks instead of fossil feedstocks, the carbon dioxide footprint will be reduced further.

Also, the embodiment achieves a simpler manufacturing process by having fasteners and interfaces integral with and made of the same material as, a shell part. This facilitates fast and simple assembly of components into the shell parts, as well as fast and simple assembly of two shell parts into a handle for an endoscope.

Further, the application of such integrated fasteners and interfaces makes it possible to reduce or even avoid the use of glue in the assembly process. This enables simpler manufacturing and recycling processes of the different materials applied in an endoscope handle.

In a second aspect the present teaching relates to a system comprising an endoscope according to the present embodiment and variations thereof, a monitor and a control unit.

In a third aspect the teaching relates to a method of assembling an endoscope. This method comprises:
- Providing a polypropylene material comprising at least 20 % bio-based polypropylene,
- Injection molding two shell parts from the polypropylene material, where the shell parts are molded with integrated fasteners configured for connecting the two shell parts to each other to form a handle of the endoscope, and, the shell parts are molded with at least one integrated interface configured to connect a shell part to at least one component selected from the group: a roller for bending the bending section by pulling a steering wire, a wire pipe fastener connecting the wire pipes to a handle shell,
- Connecting the at least one component to the at least one interface, and
- Assembling the two handle shells with the fasteners to form an internal compartment of the handle with the component connected to a shell part and positioned in the internal compartment.

Further features and combinations consistent with the present teachings will become apparent from the dependent claims and from the following detailed description. Features described in the present teaching can be combined so as to form further arrangements within the scope of the present teaching.

In this teaching, the expression "distal" is defined to be in the direction away from the user of the endoscope and toward the patient, and "proximal" is defined to be closer to the endoscope user. For the handle of the endoscope, the distal end will be the end where the insertion cord is connected, and the proximal end is the opposite end. Further, the expression "handle" encompasses a positioning interface, or interface, which functions to control the position of the insertion cord. The handle, or positioning interface, may be an interface operated by a robotic arm, or it may be a handle operated by the hand of an endoscope user.

### Brief description of the figures

The above-mentioned embodiments, features and advantages thereof will be further elucidated by the following illustrative and nonlimiting detailed description of embodiments disclosed herein with reference to the appended drawings, wherein:
Fig. 1 shows a visualization system comprising an endoscope and a monitor with a control unit.
Fig. 2A shows a variation of a handle for an endoscope.
Fig. 2B shows a different view of the handle in Fig. 2A.
Fig. 3 shows an exploded view including two handle shell parts.
Figs. 4A - 4D show detailed views of a snap fit for assembling the two handle shell parts of Fig. 3 into a handle.
Figs. 5A - 5C show detailed views of parts of interfaces from Fig. 3.
Fig. 6 shows a handle shell part provided with components being part of the endoscope.
Fig. 7 shows a shell part to match with the shell part in Fig. 6.
Fig. 8 shows a handle shell part provided with further components being part of the endoscope.
Fig. 9 shows a flow chart illustrating the process of providing and applying bio-based PP in a handle for an endoscope.
Fig. 10 shows a bending section with steering wires and wire pipes.

In the drawings, corresponding reference characters indicate corresponding parts, functions, and features throughout the several views. The drawings are not necessarily to scale, and certain features may be exaggerated in order to better illustrate and explain the disclosed embodiments. For simplicity, this teaching will focus on a two-way bending endoscope, but the teaching is relevant for, and covers, also a four-way bending endoscope.

### Detailed description

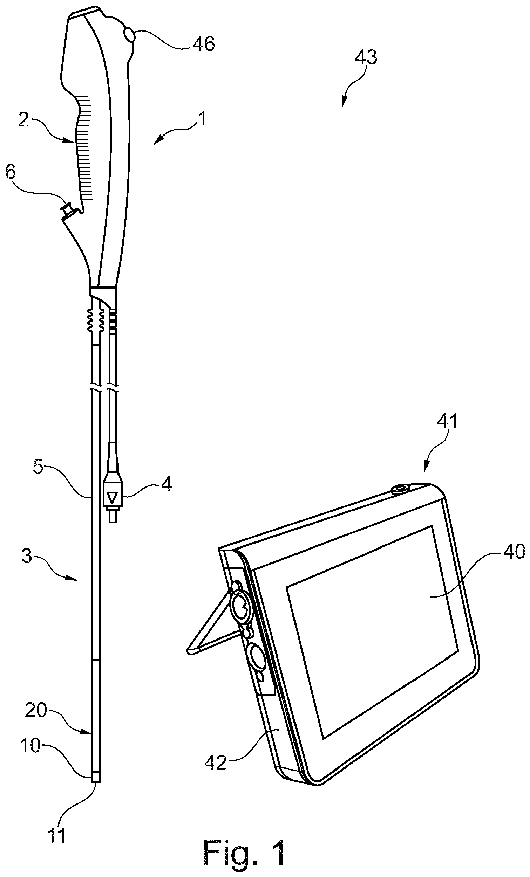

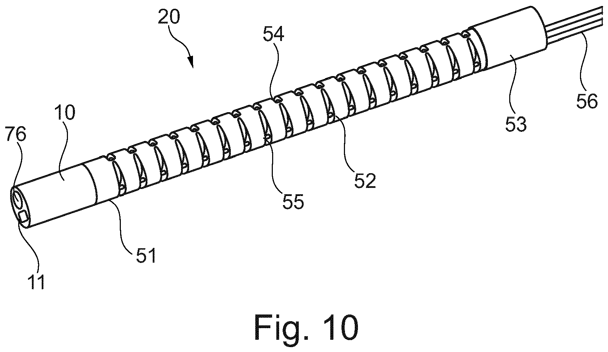

Fig. 1 illustrates a visualization system 43 comprising an endoscope 1 and a monitor 41. The endoscope 1 comprises a handle 2, an insertion cord 3 and an electrical cable with a connector 4 for connecting the endoscope 1 to a monitor 41. The insertion cord 3 is the part to be inserted into a body lumen during an endoscopic procedure. The insertion cord comprises a distal tip 10, a bending section 20 and a main tube 5. The distal tip 10 comprises a camera 11 (also shown in Fig. 10) for viewing an observation target, such as tissue, in front of the distal tip. The distal tip further comprises a light source for illuminating the observation target.

The handle 2 may comprise a port 6 to a working channel running through the insertion cord to an opening at the distal tip 10. The handle also comprises a bending lever 46, which can be used for bending the bending section 20.

The monitor 41 comprises a screen 40 and a control unit 42. The control unit 42 comprises an electronic circuit for receiving and processing the image stream from the camera 11 as well as a processor for image processing, user interface, storage of images etc. The screen 40 displays the image stream from the camera 11. The monitor 41 may be one unit or housing comprising both screen 40 and control unit 42. The screen 40 and the control unit 42 may also be separate parts, e.g., two separate units or housings (not shown).

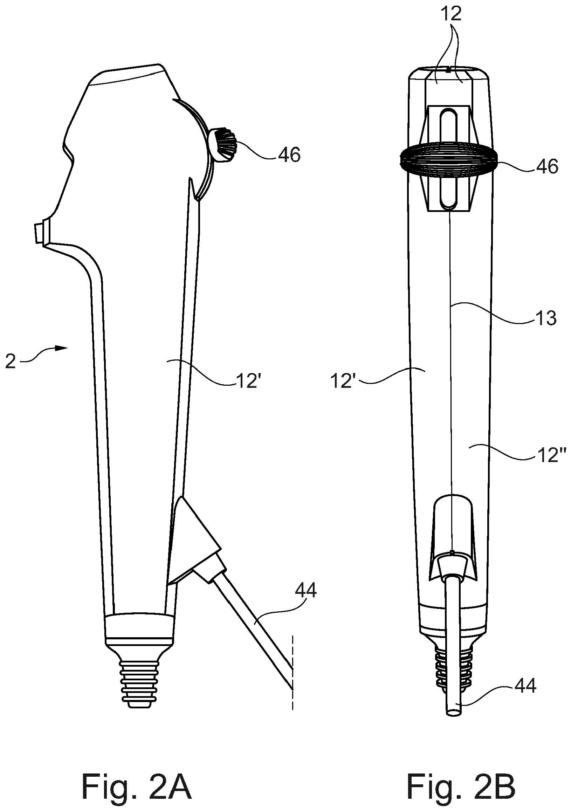

Figs. 2A and 2B show a variation of a handle for an endoscope made from two shell parts 12. The two shell parts 12 each have an outer surface. The shapes of the outer surfaces can in this variation be symmetrical in relation to a plane in which they are connected when assembled. In that case, the periphery of the handle in that plane may follow a handle split line 13 in which the two shell parts are connected.

Fig. 2A shows primarily the left shell part 12', seen from a user of the endoscope holding the handle 2 in the left hand with the left-hand thumb on the bending lever 46. When held in this way, Fig. 2B shows the endoscope seen from the back, with the left shell part 12' to the users left side and the right shell part 12" to the users' right side.

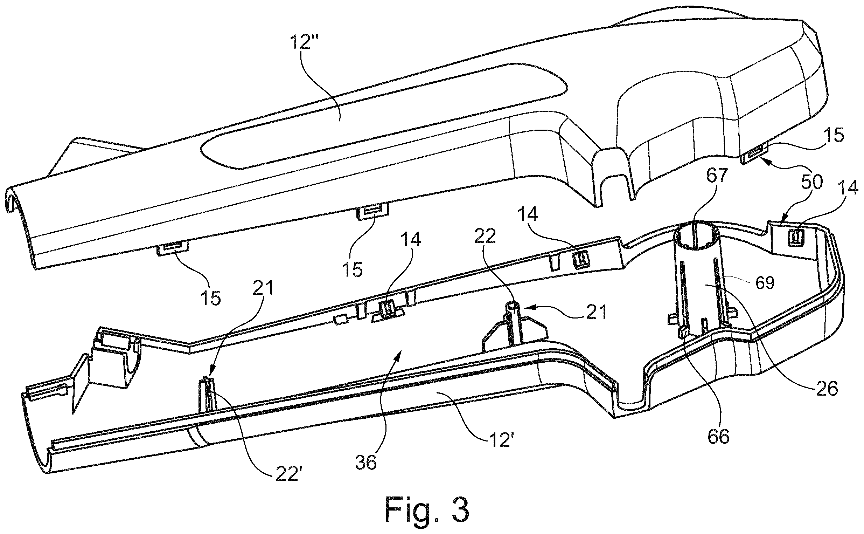

The assembled shell parts 12', 12" form an inner compartment 36 (Fig. 3). Inner surfaces of the shell parts 12 face this compartment. The inner surfaces of a shell part extend opposite to the outer surfaces of the shell part.

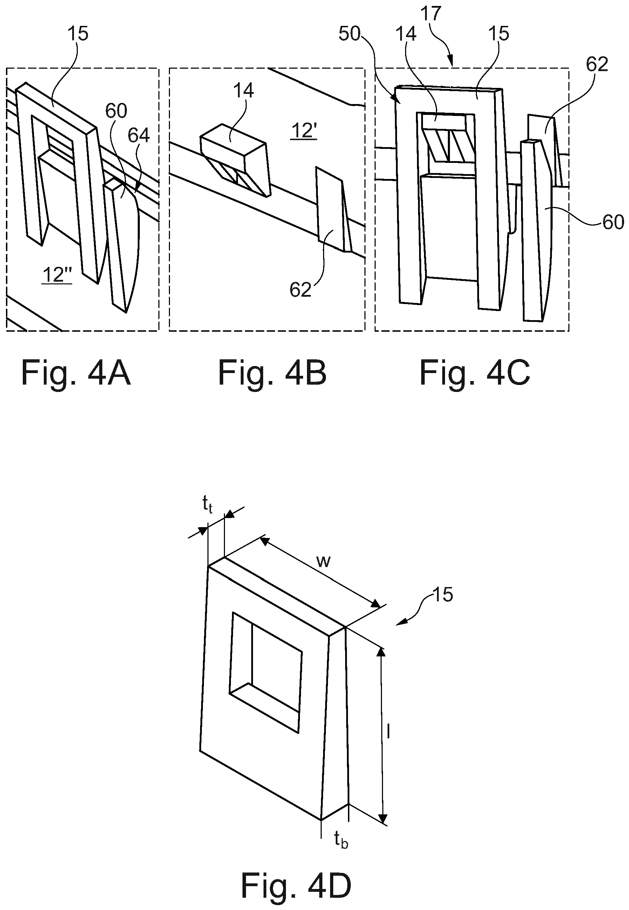

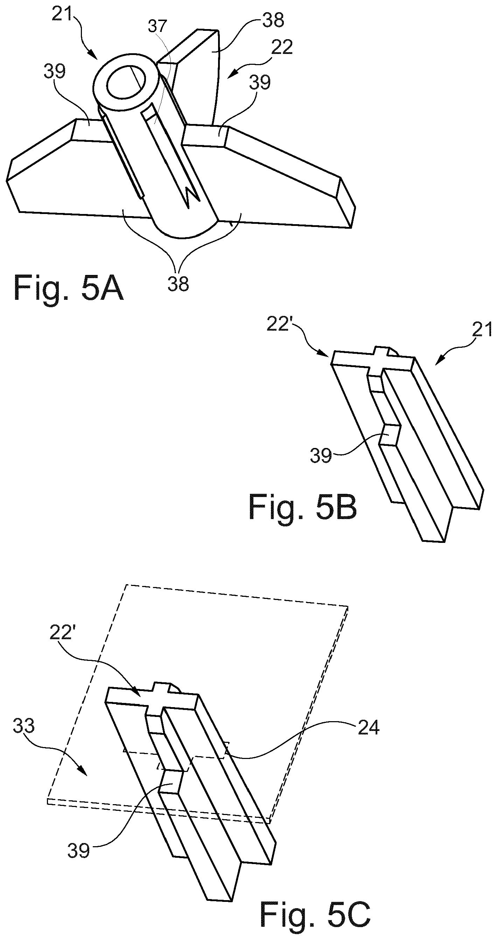

Fig. 3 shows a perspective view of a set of the shell parts 12' ,12" for forming the handle 2 of the endoscope 1. The two shells parts 12', 12" may be symmetrically shaped on their outer surfaces, but the inner surfaces of the two shell parts may differ as these can be molded with a number of interfaces 21 (also shown in Figs. 5A and 5B) and fasteners 17 (see Fig. 4) for the connection of different components of the endoscope to a shell part, and for assembly and interconnection of two or more shell parts into a handle 2. Both fasteners 17 and interfaces 21 may be integrated with the shell parts 12. As used herein "integrated" means that the integrated portion is made in one-piece with the portion into which it is integrated. Thus, a fastener integrated with a shell comprises a one-piece part including the shell and the fastener. A fastener 17 may comprise a snap fit 50 including a snap hook 14 and a snap lug 15, described below in particular with reference to Figs. 4A-4D. A snap fit fastener comprises a first feature and a second feature adapted to mate with the first feature. The features can be the snap hook 14 and the snap lug 15, in an example of the snap fit fastener.

Both fasteners 17 and interfaces 21 may be of the type snap fits, press fits, heat staking, or any other fastening method where a major part, or the entirety, of the fastener 17 or the interface 21 can be molded as an integrated part of a shell part 12. This could also involve ultrasonic welding, i.e., the fasteners or interfaces could be designed for being connected by ultrasonic welding. For the fasteners for assembly of shell parts 12 into a handle having an inner compartment 36, snap fits 50 are illustrated in Fig. 3 and 4. Here, parts for snap fits are arranged on the inner surfaces of the shell parts 12 close to the handle split line 13. If the interfaces are to be connected or secured by heat staking or ultrasonic welding, they should also be provided with a design such that they are configured for heat staking or ultrasonic welding.

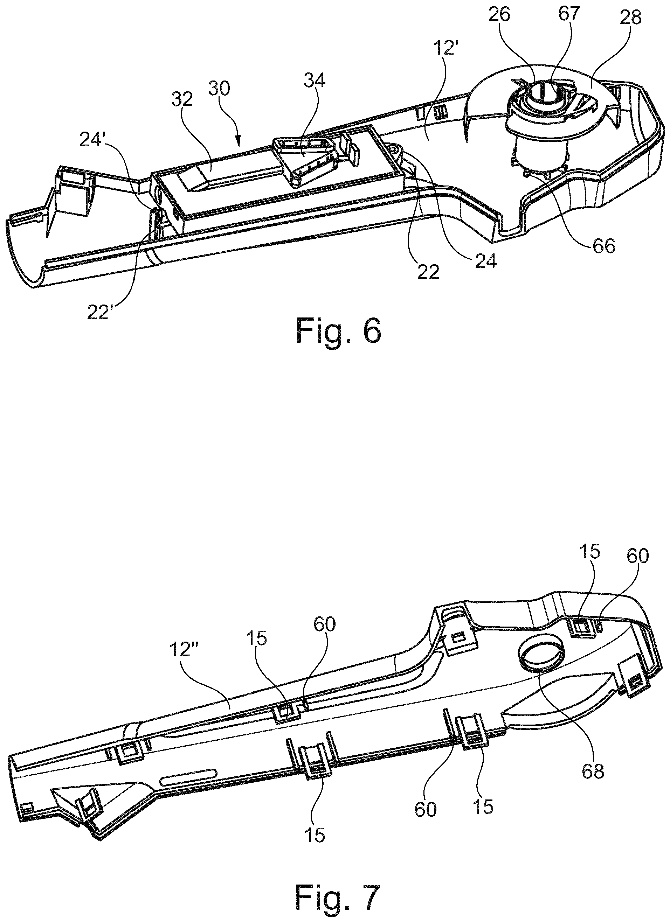

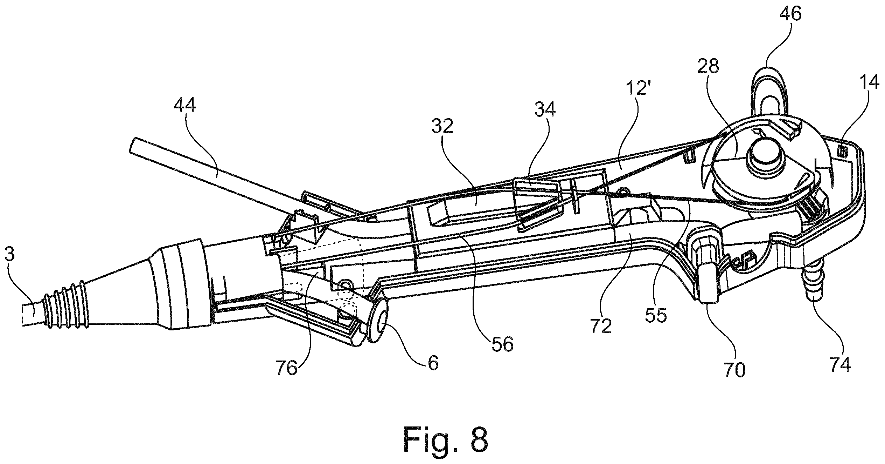

Referring to Fig. 6 and Fig. 8, the components to be arranged in the compartment 36 may be one or more of an enclosure for PCB 32, a roller 28 for bending the bending section 20 by pulling a steering wire 55, a wire pipe fastener 34 connecting wire pipes 56 to a handle shell part 12, buttons e.g., for storing an image, a working channel port 6 (or an Y-connector or biopsy connector) enabling insertion of tools into and through a working channel 76 of the endoscope, a suction valve 70 controlling suction through a working channel 76, tubing 72 for suction, e.g., a tubing connecting the suction valve 70 to the working channel 76. The suction valve 70 is also connected to a suction connector 74, which in use may be connected to a vacuum or a pump. The interfaces 21 for connecting one or more components to a shell part are an integrated part of at least one shell part and may also comprise a corresponding part on the component. The two parts are in that case configured to connect with each other.

For each snap fit 50, the snap hook 14 may be placed on one shell part, and a corresponding snap lug 15 may be placed on the other shell part. When the two shell parts 12', 12" are pressed together the snap hooks 14 will engage with the snap lugs 15 and the two shell parts will be connected. In the range of 6 -12 snap fits may be applied to provide a strong and stable connection between the two shell parts. In one variation 7 - 9 snap fits 50 are applied for connecting the two shell parts.

For some variations of fasteners or interfaces, it may be advantageous to improve the strength or stability of the interface with a glue anchor locking two parts of an interface to each other without depending on adherence to the interface parts, or at least not the part formed in one piece with a shell part 12. E.g., the two interface parts forms a space which is filled with glue, and when the glue hardens the two parts are locked together.

The snap fit parts in Fig. 3 are shown in more detail in Figs. 4A-4D, showing the snap hook 14 (Fig. 4B) and the snap lug 15 (Fig. 4A), but as an integrated part of a shell part 12, and also showing the snap hook 14 and the snap lug 15 in an assembled state (Fig. 4C). Fig. 4D shows some example dimensions for the snap lug part. In Fig. 4D the snap lug part 15 is shown as a separate part even though it may preferably be integrated with a shell part. The snap lug part 15 may have a length I from the connection to the shell part 12 to the top in the range 6-12 mm, e.g. 9 mm. The width w may be in the range 6-9 mm, e.g. 7 mm. The thickness at the bottom t_{b}, where the connection to the shell part is, may be in the range 1-3 mm, e.g. 1.6 mm. The thickness at the top tt is in the range 0.6 - 2 mm, e.g. 1 mm. The snap hook 14 may comprise a resilient protrusion which after being bent returns to its prior position. The resilient protrusion, or tab, comprises a recess (a through-hole is shown although the recess may be an indentation). During assembly the snap hook bends the tab until the snap hook at least partially penetrates the recess, allowing the tab to return to its prior position.

These dimensions have been found to provide a good balance to achieve both the flexibility for connecting with the snap hook 14 in a simple and easy way and the strength to maintain a strong and robust connection between the two shell parts 12', 12". For comparison the average thickness of a shell part 12 may be in the range 1-4 mm, maybe in the range 1.5 - 2.5 mm.

Alignment means may be positioned along the line of assembly, i.e., the handle split line 13 as described below with reference to Fig. 4. The alignment means may be placed close to the snap fit parts but do not need to be. The alignment means ensure that the outer surface of the assembled handle is as smooth as possible without any significant edge in the transition from one shell part to the other. The alignment means may be made from a first alignment part 60 and a second alignment part 62. From Fig. 4A it is seen that the first alignment part 60 and a part of the shell part 12", together form a U-shaped receptacle space 64 with dimensions configured to receive a second alignment part 62 integrated with the other shell part 12'. The number of alignment means, i.e., pairs of a first alignment part 60 and a second alignment part 62, may be in the range of 5 - 13, maybe 6 - 10. The combination of the snap fit 50 connections and the alignment means has the effect that when the two shell parts are snapped together, they cannot be displaced relative to each other in any direction. Also, along the handle split line 13 the connection will be firm and robust. By preventing displacement, it is also prevented that the shells will un-snap and separate.

The interfaces 21 can be the press fit rods 22 shown in Fig. 3 for connecting a printed circuit board (PCB) 33 (shown in Fig. 5C) and/or a platform 30 holding the printed circuit board (PCB) 33 and/or for holding a wire pipe fastener 34 (see Fig. 6). This platform 30 can have the form of a box or an enclosure 32 for providing a watertight sealing for the PCB 33. The wire pipe fastener 34 may be placed on an outer surface of this box or enclosure 32. There can be several different parts of the interface 21 connecting this platform to the handle shells. This may include snap fits between the platform and a handle shell. It may also include press fits in relation to one or both shell parts 12', 12". A plurality of interfaces 21 may provide alternative or additional alignment means since they may prevent relative movement between the shell parts, particularly when positioned near the edges of the shell parts.

A press fit fastener may have a stud or rod 22 and a cavity. An example can be seen in Fig. 6, where the rods 22, 22', extending from a shell part 12', are in engagement with cavities or press fit receivers 24, 24' connected to the enclosure 32.

Examples of interfaces are shown in Figs. 5A-5C. Figs. 5A and 5B show two examples of press fit rods 22, 22', for connecting the platform 30 to a shell part 12. Both rods 22, 22' are extending from a shell part 12 (not shown in Fig. 5), and the platform 30 is provided with press fit receiver means 24, 24' (shown in Fig. 6 as counterparts to the interfaces 22, 22'), which can be holes or slits for receiving a press fit rod 22. The press fit rods 22, 22' are provided with support walls 38, for increased stability and stiffness of the connection, and with support surfaces 39 for controlling correct positioning of the platform 30 in the compartment 36 and in relation to the shell part 12. The press fit rods may also be provided with interface ribs 37 extending from a core. This will improve the strength of the press fit connection. Fig. 5C shows a PCB 33 supported directly by an interface 22'. The PCB may also be arranged in the enclosure 32 shown in Fig. 6.

The platform 30 or another component in the compartment 36 of the handle can comprise interfaces to both shell parts 12', 12", which provides a more mechanically stable and strong handle, providing a firm feeling of the handle to the endoscope user. As illustrated in Fig. 5A and 5B the two interfaces 22, 22' may have a different design adapted to fit each end of the enclosure 32 for the PCB as illustrated in Fig. 6.

In a variation the platform 30 may have the form of a frame to which several components are connected. The frame may be connected to one or two shell parts through interfaces and/or fasteners.

Fig. 6 shows a handle shell with a roller 28, an enclosure 32 for a printed circuit board (PCB), and a wire pipe fastener 34 arranged on a surface of the enclosure 32 for the PCB. The wire pipe fastener 34 may be molded as part of this surface, and thereby be an integrated part of the enclosure 32. The interfaces can also be the roller interface 26 which is an axle, molded as part of a shell part 12', to which the roller 28 is rotatably mounted. Steering wires 55 (Fig. 8 and Fig. 10) are mounted to the roller 28 such that a rotational movement of the roller 28 around the roller interface 26, may pull one of two steering wires 55 and thereby bend the bending section 20, illustrated in Fig. 10. Fig. 8 illustrates the wire pipes 56 and how the wire pipes 56 are connected to the wire pipe fastener 34.

It is illustrated in Fig. 7 that the other shell part 12" has structure on the inner surface to limit movement of the roller in an axial direction of the axle, without obstructing rotation of the roller 28. This structure may be a circular wall 68 projecting from the inner surface, surrounding a space into which the roller interface 26 axle fits when the two shell parts 12', 12" are assembled. The circular wall 68 is shown in Fig. 7. The height of the circular wall 68 projecting from the inner surface can be adjusted to limit movement of the roller 28 in the axial direction of the roller interface 26 axle. The height of the circular wall could be in the range 3 - 4 mm, and the length of the roller interface 26 in the axial direction could be 20 - 30 mm, maybe 23 - 25 mm. The length of the roller part to receive the roller interface 26, may be 19 - 21 mm along the axis of rotation of the roller 28.

As indicated in Fig. 3 and in Fig. 6 the roller interface 26, forming an axle for the roller 28, may have ribs 66, 67 for enforcing the connection to the shell part 12'. There are external ribs 66 on the outside of the roller interface 26 and in the zone where the roller interface 26 is connected with the shell part 12'. The external ribs 66 may also function as a support for the roller 28, e.g., by having a flat surface of contact to the roller, i.e. the surface opposite to the shell part 12'. In an example there may be 7 - 9 external ribs 66, which may have a thickness, in a direction following the periphery of the roller interface 26, in the range 0.8 - 1.2 mm, e.g., 0.9 - 1.1 mm. The external ribs 66 could have a length, in a direction pointing away from a center axis of the roller interface 26, in the range 1.7 - 2.4 mm, e.g., 1.9 - 2.2 mm. The external ribs 66 could have a height, in a direction pointing away from the shell part 12' to which it is connected, in the range 1.7 - 2.3 mm, e.g., 1.9 - 2.1 mm.

Also, the external ribs 66 may be connected to rib extensions 69 continuing from the external rib 66 in a direction pointing away from the shell part 12' in an axial direction of the roller interface 26. The rib extensions 69 may reduce the friction of the roller 26 when this is rotated by the bending lever 46.

The inner side of the roller interface 26 may have internal ribs 67. The internal ribs may extend from the shell part 12' through the roller interface 26 in the axial direction. Thereby, the internal ribs may both enforce the connection between the roller interface 26 and the shell part 12' and improve the strength and stiffness of the roller interface 26. In an example there may be 4 - 8 internal ribs 67, which may have a length, in a direction perpendicular to a center axis of the roller interface 26, in the range 0.4 - 0.6 mm. The internal ribs 67 may have a thickness, in a direction following the periphery of the roller interface 26, which may be 1.1 - 1.6 mm at the end where the internal ribs are connected to the shell part 12'. This thickness may be gradually reduced when moving towards the other end of the roller interface in the axial direction of the roller interface 26. At the end opposite the end where the roller interface is fixedly connected, e.g., molded in on piece with, the handle shell 12', the thickness of the internal ribs 67 may be in the range 0.4 - 0.6 mm. By having the ribs 66, 67 it is possible to make the roller interface with smaller dimensions, such as a material thickness in the range 0.4 - 0.6 mm. This will overall save on the amount of material to be applied.

The above-described solutions related to fasteners 17 and interfaces 21 enable that the shell parts 12', 12" can be assembled to form the handle 2 of the endoscope 1 and connecting the shell parts 12', 12" to each other by the fasteners 17, without any non-polypropylene material providing strength or stability to the connection. The same may apply for the connection of different components to a shell part 12 by interfaces 21. These interfaces 21 may also be without non-polypropylene material being applied for providing strength or stability to the connection. For both the fasteners 17 and the interfaces 21 this means that the use of glue or adhesives can be avoided. This has the effect of enabling a simpler manufacturing since gluing processes can be avoided. The effect is larger when the shell parts 12', 12" are made from PP, as special glue may be needed for PP and also more specialized gluing processes may be needed for PP.

To provide strength and stability to a connection, is, in the present disclosure understood to mean that without the part or material the connection would not work as intended in a reliable way, e.g., the parts could be unintentionally separated, or the parts could move relatively to each other in a way which could compromise the functionality of the endoscope.

In one variation the fasteners and/or the interfaces comprise only parts which are integrated with a shell part or with a component to be secured in the handle compartment.

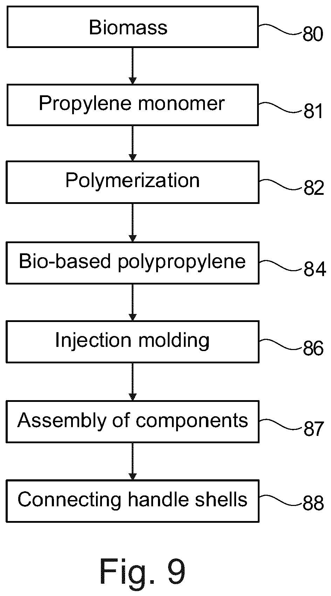

Fig. 9 shows a flowchart giving an overview of major steps in the process of providing and applying bio-based polypropylene for endoscope handles 2. A first step 80 in this process, may be to provide the biomass needed to produce the bio-based PP.

In this disclosure bio-based PP is understood to originate from biomass, which is renewable, e.g., within a time span of up to 10 years or up to 50 years. This is opposed to so-called synthetic PP which is made from a fossil-based feedstock, such as crude oil, which is not renewable, at least not when considering a time span of up to e.g., a thousand years.

One way to establish whether the PP is bio-based or based on a fossil feedstock, is by a carbon 14 analysis. A carbon 14 analysis can also provide an estimate of the relationship between the content of bio-based PP and the content of fossil-based PP.

Bio-based polypropylene (PP) may be manufactured from natural materials such as lignocellulosic biomass (starch and cellulose), fatty acids, and organic waste, e.g., food waste or waste products from farming or forestry. Also, corn, sugar cane, vegetable oil, and some other types of biomass may be applied.

A second step 81 in the process may be to provide propylene monomer from the biomass. There are different methods for producing bio-based propylene, which is the starting monomer for PP. The PP is made from propylene by polymerization. The method selected for producing propylene may depend on the organic raw materials. For some raw materials, such as sugar and starch, fermentation may be a relevant step in providing the propylene. For some types of biomass, gasification may be a relevant step in providing the propylene. In general, gasification may be applied for a larger range of biomass sources, but fermentation is a cheaper and less energy consuming process.

A third step 82 is the polymerization of propylene monomers into polypropylene, e.g., by a chain-growth polymerization. This provides the bio-based polypropylene in step four 84, which in step five 86 is applied for injection molding of the handle shell parts 12 including the integrated fasteners 17 and interfaces 21.

In step six 87 the different components, such as enclosure for PCB 32, wire pipe fastener 34, a roller 28, suction valve, tubing for suction, buttons etc., are arranged in one shell part and fastened by interfaces being integrated in the shell part. Further, steering wires and wire pipes, as well as cables and PCB may be arranged and the insertion cord and a working channel may be connected, before in step seven 88 clicking the two handle shell parts 12', 12" together to form the handle 2 with the components in the compartment formed by the shell parts.

Some changes in the design of the endoscope handle have in this teaching been found to simplify the manufacturing of the endoscope handle and has enabled a shift from previous used polymers for the handle shells, such as ABS, into the use of PP, including bio-based PP.

Recycled PP may comprise a combination of bio-based PP and fossil-based PP. As more and more bio-based PP is applied, the fraction of bio-based PP in recycled PP will increase.

The content of bio-based PP does not influence the use of additives which may be added to the PP in order to provide specific, e.g., mechanical, characteristics to the endoscope handle material.

The PP of the shell parts may comprise at least 20 % bio-based PP, alternatively, at least 40 % bio-based polypropylene. The fraction of bio-based PP may also be at least 60 %, or at least 80 %. A higher fraction of bio-based PP in the handle shell, means that the carbon dioxide emissions related to the endoscope handles during their life cycle, will be lower. The shell parts may consist essentially of PP. Processing additives may be added as is known in the art. Alternatively, the shell parts may comprise at least 60% PP, preferably more than 70% PP, of which at least 20% is bio-based PP. Fibers, such as natural fibers, may be added to modify physical properties, such as strength. In one variation, the shell parts may be overmolded to provide a desired texture feel or to provide color to portions of the handle. The overmolding material may or may not be PP.

One practical way to add bio-based PP as a fraction of the handle shell material is to apply the so-called bio-attributed concept, where the bio-based fraction of the PP is calculated from the mass balance approach (or model). This means that the fraction of bio-based PP in each handle shell may not be known, but the average fraction over some time, or for a certain number of handle shells, is well known. The supplier of the PP raw material to the molding, will comply with the relevant standards (such as the series of ISO 16620 standards: ISO 16620-1:2015, ISO 16620-2:2019, ISO 16620-3:2015, ISO 16620-4:2016, ISO 16620-5:2017) related to the mass balance model. Applying this approach means that in order to know the bio-based fraction of PP in a specific handle shell, or a specific batch of handle shells, an analysis, such as an analysis of the carbon-14 content, may be necessary. The rest of the PP may come from fossil resources. Some of the PP could also be based on recycled PP.

Fig. 10 shows a distal end of the insertion cord 3 of an endoscope 1, where the bending cover (not shown) typically covering the bending section 20 part has been removed. The proximal end of the bending section is attached to the main tube 5, and the distal end of the bending section is attached to the distal tip 10. In this example the bending section is molded in one piece and comprises a number of segments 52. A proximal end segment 53 is connected to the distal end of the main tube 5. A distal end segment 51 is connected to the distal tip 10. The segments are held together by hinges 54, so that the segments can be bent relative to each other by manipulation of the steering wires 55.

Steering wires 55 are connected in a fixed connection to the distal end, e.g., the distal end bending segment 51. I.e., the steering wire is preferably not movable in relation to the distal end bending segment. Between the handle and the distal end of the main tube 5, or the proximal end of the bending section 20, the steering wire 55 is guided in a wire pipe 56 which is secured to the wire pipe fastener 34 in the handle 2. The steering wires 55 are connected to the roller 28 in the handle 2. Other designs of the bending section are also possible.

### List of references

| | | | |
|---|---|---|---|
| 1 | endoscope | 38 | support wall |
| 2 | handle | 39 | contact surface |
| 3 | insertion cord | 40 | screen |
| 4 | electrical cable with plug | 41 | monitor |
| 5 | main tube | 42 | control unit |
| 6 | working channel port | 43 | visualization system |
| 10 | distal tip | 44 | video cable |
| 11 | camera | 46 | bending lever |
| 12,12', 12" | shell part | 50 | snap fit |
| 13 | handle split line | 52 | segment |
| 14 | snap hook | 54 | hinge |
| 15 | snap lug | 55 | steering wire |
| 17 | fastener | 56 | wire pipe |
| 20 | bending section | 60 | first alignment part |
| 21 | interface | 62 | second alignment part |
| 22 | press fit rod | 64 | receptacle space |
| 24 | press fit receiver | 66 | external ribs |
| 26 | roller interface | 67 | internal ribs |
| 28 | roller | 68 | circular wall |
| 30 | platform | 69 | rib extension |
| 32 | enclosure for PCB | 70 | suction valve |
| 33 | PCB | 72 | suction tube |
| 34 | wire pipe fastener | 74 | suction connector |
| 36 | compartment | 76 | working channel |
| 37 | interface rib | | |

## Claims

1. An endoscope comprising a handle and an insertion cord with a bending section which is bendable by manipulation of steering wires movably arranged in wire pipes, the handle comprising:
- two shell parts assembled with fasteners, the fasteners made of the same material and in one-piece with one or another of the two shell parts, the assembled shell parts forming a compartment enclosed by the shell parts,
- at least one component selected from a group comprising: a roller for bending the bending section by pulling a steering wire, a wire pipe fastener connecting the wire pipes to one of the shell parts, an enclosure for a printed circuit board, and
- interfaces configured to secure the at least one component to at least one of the two shell parts inside the compartment, at least one of the interfaces made of the same material and in one-piece with the one or the other of the two shell parts, the interfaces extending into the compartment,
the shell parts being made from polypropylene, wherein the polypropylene comprises bio-based polypropylene.

2. The endoscope of claim 1, wherein the polypropylene comprises at least 20 % bio-based polypropylene, alternatively, at least 40 % bio-based polypropylene.

3. The endoscope according to any one of the preceding claims, wherein the fasteners comprise snap fit fasteners or press fit fasteners.

4. The endoscope according to claim 3, wherein 6 -12 snap fits are applied to connect the two shell parts.

5. The endoscope according to any one of the preceding claims, wherein both a roller for bending the bending section by pulling one of the steering wires, and a wire pipe fastener connecting the wire pipes to one of the shell parts, are secured to one of the shell parts inside the compartment via interfaces being integral with and made in the same material as a shell part.

6. The endoscope according to any one of the preceding claims, wherein at least one further component selected from the group: a working channel port enabling insertion of tools into and through a working channel of the endoscope, a suction valve controlling suction through a working channel of the endoscope, are secured to at least one of the shell parts inside the compartment via interfaces being integral with and made in the same material as the at least one shell part.

7. The endoscope according to any one of the preceding claims, wherein at least one interface is configured for heat staking or ultrasonic welding.

8. The endoscope according to any one of the preceding claims, wherein the shell parts are connected to form the handle, and the shell parts are connected to each other by the fasteners, without any non-polypropylene material adding strength or stability to the connection.

9. The endoscope according to any one of the preceding claims, wherein the components are connected to at least one of the shell part by the interfaces without any non-polypropylene material adding strength or stability to the connection.

10. The endoscope according to any one of the preceding claims, wherein a roller is rotatably connected to a roller interface forming an axle for the roller, the roller interface being made of the same material and in one-piece with one of the shell parts, and ribs being provided to enforce a connection between the roller interface and the shell part.

11. A system comprising an endoscope according to any one of claims 1-10, a monitor and a control unit.

12. Method of assembling an endoscope comprising:
- providing a polypropylene material comprising at least 20 % bio-based polypropylene,
- injection molding two shell parts from the polypropylene material, where the shell parts are molded with integrated fasteners configured to connecting the two shell parts to each other to form a handle of the endoscope, and, at least one of the shell parts are molded with at least one integrated interface configured to connect a shell part to at least one component selected from the group: a roller for bending the bending section by pulling a steering wire, a wire pipe fastener connecting the wire pipes to a handle shell, an enclosure for a printed circuit board,
- connecting the at least one component to a shell part by application of the at least one interface,
- assembling the two shell parts by the fasteners thereby forming the handle having an internal compartment inside which the component is connected to a shell part.
